# EUROPÄISCHE PATENTSCHRIFT

(11) **EP 1 893 773 B1**
(45) Veröffentlichungstag und Bekanntmachung des Hinweises auf die Patenterteilung: **23.01.2013**
(21) Anmeldenummer: 06754507.9
(22) Anmeldetag: 22.06.2006
(51) Int. Cl.: C12Q 1/68, C07H 21/00

(54) **NUKLEOLIPIDE UND DEREN VERWENDUNG, SOWIE VORRICHTUNGEN ZUR NUKLEINSÄUREANALYTIK**
NUCLEOLIPIDS AND USE THEREOF, AND DEVICES FOR NUCLEIC ACID ANALYSIS
NUCLEOLIPIDES ET LEUR UTILISATION AINSI QUE DISPOSITIFS D'ANALYSE D'ACIDES NUCLEIQUES

(30) Priorität: 22.06.2005 EP 05013432
(43) Veröffentlichungstag der Anmeldung: 05.03.2008
(73) Patentinhaber: Universität Osnabrück, 49074 Osnabrück (DE)
(72) Erfinder: ROSEMEYER, Helmut, 49076 Osnabrück (DE)
(74) Vertreter: Kröncke, Rolf
(86) Internationale Anmeldenummer: PCT/EP2006/006013
(87) Internationale Veröffentlichungsnummer: WO 2006/136411

(56) Entgegenhaltungen:
- MIAO W, DU X, LIANG Y: "Molecular Recognition of Nucleoliplid Monolayers of 1-(2-Octadecyloxycarbonylethyl)cytosine to Guanosine at the Air-water Interface and Langmuir-Blodgett Films" LANGMUIR, Bd. 19, 17. Mai 2003 (2003-05-17), Seiten 5389-5396, XP002351354
- HUANG J ET AL: "Molecular recognition of nucleolipid amphiphile octadecanoyl ester of 1-(2-carboxyethyl) adenine to the complementary nucleobases. Part I: characterization of the monolayer behavior at the air/water interface and photodimerization in the Langmuir-Blodgett film matrix under ultraviolet irradiation" THIN SOLID FILMS, ELSEVIER-SEQUOIA S.A. LAUSANNE, CH, Bd. 326, Nr. 1-2, 4. August 1998 (1998-08-04), Seiten 217-222, XP004141487 ISSN: 0040-6090
- ROSEMEYER H ET AL: "A NUCLEOLIPID WITH ANTIVIRAL ACYCLOGUANOSINE AS HEAD GROUP SYNTHESIS AND LIPOSOME FORMATION" ANGEWANDTE CHEMIE INTERNATIONAL EDITION IN ENGLISH, Bd. 24, Nr. 6, 1985, Seiten 501-502, XP002351355 ISSN: 0570-0833
- DAVIS F ET AL: "Structured thin films as functional components within biosensors" BIOSENSORS & BIOELECTRONICS, ELSEVIER SCIENCE PUBLISHERS, BARKING, GB, Bd. 21, Nr. 1, 18. November 2004 (2004-11-18), Seiten 1-20, XP004943498 ISSN: 0956-5663

## Beschreibung

Gegenstand der Erfindung ist ein Verfahren zur Isolierung und/oder Identifizierung von bekannten oder unbekannten, gegebenenfalls mit Reportergruppe markierten Nukleinsäuresequenzen (Targetsequenzen) durch basenspezifische Hybridisierung mit im wesentlichen komplementären Sequenzen ( im Folgenden als Probenoligonukleotide, Probensequenzen oder Probennukleinsäuren bezeichnet), die zu einer Bibliothek von Sequenzen gehören. Die Probensequenzen sind dadurch charakterisiert, dass sie alle an einem ihrer Termini (5'-Ende oder 3'-Ende, vorzugsweise am 5'-Ende) ein ein-, doppel- oder mehrkettigen Lipidanteil tragen, der eine Spreitung in monomolekularer Schicht an einer Flüssigkeits-Gas oder Flüssig-Flüssig Phasengrenzfläche ermöglicht.

### Hintergrund der Erfindung

Nukleinsäuren haben als Träger bzw. Überträger der genetischen Information eine zentrale Bedeutung in der belebten Natur. Mit der zunehmenden Kenntnis der grundlegenden molekularbiologischen Mechanismen ist es in den letzten Jahren möglich geworden, die Neukombination von Genen zu betreiben. Diese Technologie eröffnet z.B. neue Möglichkeiten in der medizinischen Diagnostik und Therapie sowie in der Pflanzenzüchtung.

Ein wichtiges Werkzeug zur Erklärung dieser Zusammenhänge und zur Lösung der Probleme war und ist der Nachweis von Nukleinsäuren und Nukleinsäurefragmenten, und zwar sowohl was ihren spezifischen Nachweis betrifft, als auch was ihre Sequenz, also ihre Primärstruktur angeht. Weiterhin ist ein Bestandteil molekularbiologischen Arbeitens die Isolierung von Nukleinsäuresequenzen, z.B. im Rahmen von Aufreinigung und Weiterbearbeitung ausgewählter Nukleinsäuren.

Die spezifische Nachweisbarkeit von Nukleinsäuren beruht auf der Eigenschaft der Moleküle, mit anderen Nukleinsäuren durch Ausbildung von Basenpaaren über Wasserstoffbrückenbindungen in Wechselwirkung zu treten - zu "hybridisieren". Die Analyse von Genen bzw. Genabschnitten erfolgt heute auf DNA-Chips. Solche Chips, Arrays oder DNA-Microarrays bestehen aus einem festen Träger (z.B. einem Glasobjektträger), auf dem in einem regelmäßigen Muster einzelsträngige DNA-Moleküle bekannter Sequenz aufgebracht sind. Die Herstellung derartiger Nukleinsäurechips erfolgt entweder (i) durch direkte Synthese mittels Masken und eines photolithographischen Verfahrens auf dem festen Träger, oder (ii) es werden vorgefertigte und terminal funktionalisierte Nukleinsäureproben auf aktivierten Oberflächen chemisch kovalent fixiert. Die DNA-Analyse auf dem Chip umfasst mehrere Teilschritte: (i) Probenvorbereitung (Extraktion, PCR etc.); (ii) Hybridisierung auf dem Chip; (iii) stringentes Waschen; (iv) Detektion; (v) bioinformatische Auswertung.

Beide Verfahren zur Herstellung von DNA-Chips sind mit zahlreichen Problemen behaftet. Bei dem 1. Verfahren findet die Oligonukleotidsynthese direkt auf dem Träger einschließlich umfangreichen Entschützungsreaktionen und Waschschritte statt. Dieses stellt ein sehr aufwendiges Verfahren dar, insbesondere wenn der Array eine Vielzahl von unterschiedlichen Nukleinsäureproben umfasst. Beim 2. Verfahren muss beispielsweise die feste Oberfläche - üblicherweise eine Glasplatte - auf komplizierte Weise mit funktionellen Gruppen aktiviert werden. Erst dann ist das Aufbringen von ebenfalls funktionalisierten vorgefertigten Nukleinsäuren bekannter Sequenz möglich. Dieses Aufbringen ("Spotten") ist ebenfalls problembehaftet und bedarf teurer und komplizierter Geräte, der sogenannten "Microarrayer". Anschließend erfolgt eine chemische Umsetzung zwischen den funktionalisierten vorgefertigten Nukleinsäuren und den aktivierten funktionellen Gruppen an der Oberfläche des Arrays, um eine kovalente Bindung zwischen Array und Nukleinsäure zu erreichen.

Zur Isolierung von Nukleinsäuren wird heutzutage üblicherweise ein Verfahren unter Verwendung von chaotropen Verbindungen in Kombination mit soliden Materialien, wie Silika oder Silikaderivate oder Magnetpartikel eingesetzt. Der Nachteil dieser Verfahren ist ein aufwendiges Isolierungsverfahren mit vielen Wasch- und Zentrifugationsschritten.

Die Isolierung von mRNA über OligodT-Nukleotide erfordert weiterhin eine Vorbehandlung der soliden Materialien, entweder um eine direkte kovalente Kopplung oder um eine indirekte Kopplung der OligodT-Moleküle an die soliden Materialien zu ermöglichen.

Eine weitere Alternative ist eine Aufreinigung mit Hilfe von Membranen.

Eine Aufgabe der vorliegenden Erfindung ist die Bereitstellung eines neuen, erheblich einfacheren und preiswerteren Verfahren zur Analytik von Nukleinsäuren, die aufwendige chemische Kopplungsreaktionen mit den Trägermaterialien vermeidet.

Eine weitere Aufgabe der vorliegenden Erfindung ist die Bereitstellung neuer Verbindungen, die insbesondere in Verfahren zur Analyse von Nukleinsäuren verwendet werden können.

### Zusammenfassung der Erfindung

Die vorliegende Erfindung stellt Verfahren zur Herstellung von Nukleolipiden bereit, die einen lipophilen Anteil (auch als Lipid bezeichnet) und ein Nukleosid- bzw. Oligo- oder Polynukleotidanteil (im Folgenden auch als Nukleinsäureanteil bezeichnet) umfassen. Diese beiden Komponenten sind über einen Linker (z.B. enthaltend ein Stickstoffatom), einen Abstandshalter (auch als Spacer bezeichnet) und eine funktionelle Einheit, die auch als Konnektor bezeichnet wird (z.B. einen Phosphorsäurediesteranteil), miteinander verbunden. Der Konnektor ist dabei eine Phosphorsäurediesterverbindung, die zwischen dem Lipidanteil und dem Nukleotidanteil ausgebildet wird, wenn eine Phosphoramiditgruppe einer Verbindung mit lipophilem Anteil und funktionellem Anteil mit der 3' oder 5' OH-Gruppe eines Nukleosids, z.B. dem 3' oder 5' Ende eines Oligonukleotids oder Polynukleotids, umgesetzt wird. Weiterhin stellt die vorliegende Erfindung Verbindungen bereit, die als Ausgangsverbindungen für die Herstellung der oben genannten Nukleolipide geeignet sind. Diese Verbindungen, im Folgenden auch als reaktive Lipide bezeichnet, umfassen einen lipophilen Anteil und einen funktionellen Anteil, die über einen Linker und einen Abstandshalter miteinander verbunden sind, dadurch gekennzeichnet, dass der Linker eine Amingruppe oder ein Molekül mit mindestens zwei funktionellen Gruppen und mit dem lipophilen Anteil direkt verbunden ist und der funktionelle Anteil eine Phosphoramiditgruppe ist, wobei der funktionelle Anteil über ein Sauerstoffatom mit dem Abstandshalter, der mindestens eine Kohlenstoff enthaltende Gruppe umfasst, direkt verbunden ist. Weiterhin stellt die vorliegende Erfindung Verfahren zur Nukleinsäureanalytik bereit mit einer Vorrichtung, umfassend einen unteren Teil, der eine flüssige Phase aufnehmen kann und einen oberen Teil, der nicht dauerhaft mit dem unteren Teil verbunden und in den unteren Teil einsetzbar ist, wobei der obere Teil mindestens zwei voneinander abgetrennte Kompartimente aufweist und diese Kompartimente von der Oberbis zur Unterseite des oberen Teils ausgebildet sind. Das Verfahren umfasst weiterhin die Verwendung von Nukleolipiden, insbesondere Nukleolipide gemäß der vorliegenden Erfindung, die in den Kompartimenten an der Phasengrenzfläche gespreitet werden.

Schließlich betrifft die vorliegende Erfindung Verfahren zur Isolierung von Nukleinsäuren aus Proben bzw. Verfahren zum Nachweis von Nukleinsäuren in Proben.

### Kurze Beschreibung der Abbildungen

Die Figur 1 zeigt schematisch den Aufbau der erfindungsgemäßen Verbindungen. In (A) ist die erfindungsgemäße Verbindung, das reaktive Lipid, umfassend einen oder mehrere lipophile Anteile (Lipid oder im Folgenden auch F bezeichnet), den funktionellen Anteil (P) und der dazwischen liegende Linker (L) und Abstandshalter (S) gezeigt. Dargestellt sind verschiedene Ausführungsformen der reaktiven Lipide, bei denen sowohl ein- als auch doppel- oder mehrkettige Lipide in den Verbindungen vorhanden sind.

Die Figur 1 (B) stellt die Nukleolipide dar. Auch hier sind verschiedene Ausführungsformen der Nukleolipide dargestellt, bei denen sowohl ein- als auch doppel- oder mehrkettige Lipidanteil(e) in den Verbindungen vorhanden sind. Der Konnektor (K) ist dabei der aus der funktionellen Einheit verbleibende Teil, der das Nukleotid mit dem Abstandshalter, der auch als Spacer bezeichnet wird, und über den Spacer mit dem lipophilen Anteil verbindet.

Die Figur 2(A) stellt den Syntheseweg eines erfindungsgemäßen reaktiven Lipids, hier eines Phosphoramiditderivats, dar, siehe auch Beispiel 1. (B) stellt erfindungsgemäß verzweigte reaktive Lipide dar (Verbindungen 4 + 5)

Figur 3 stellt schematisch eine Vorrichtung zur DNA-Analyse dar.

Die Abbildung 4 zeigt schematisch die Funktionsweise der Vorrichtung und des erfindungsgemäßen Verfahrens zum Nachweis von spezifischen Sequenzen (Target-Nukleinsäuren) in einer Probe.

Figur 5 zeigt schematisch einen Aufbau einer Vorrichtung zur Isolierung von Hybridiserungsprodukten von Targetsequenzen und Nukleolipiden mit Hilfe eines Dip Coaters mit Wilhelmy-Plättchen.

### Detaillierte Beschreibung der Erfindung

Die vorliegende Erfindung betrifft Zwischenprodukte zur Herstellung von Nukleolipiden. Unter einem Nukleolipid wird eine chemische Verbindung mit einem Nukleosid, einem Nukleotid oder einer Nukleobase und einem ein-, doppel- oder mehrkettigen Lipid verstanden. Das oberflächenaktive Verhalten derartiger "chimärer" Moleküle wurde üblicherweise in Langmuir-Blodgett Apparaturen untersucht. Es hat sich gezeigt, dass derartige Verbindungen sich an der Phasengrenzfläche so orientieren, dass der hydrophobe Molekülteil (Lipid) in die Gasphase weist, die hydrophile Kopfgruppe hingegen in die flüssige Subphase.

Die allgemeine Synthese von Nukleolipiden und ihr Spreitungsverhalten in der Phasengrenze Flüssigkeit-Gas ist im Stand der Technik beschrieben. In der vorliegenden Anmeldung werden neue Nukleolipide beschrieben, die besonders gut zur Nukleinsäureanalytik geeignet sind.

Die Nukleolipide umfassen einen lipophilen Anteil und einen funktionellen Anteil, die über einen Linker und einen Abstandshalter miteinander verbunden sind, sowie einen Nukleosid-, Oligonukleotid- oder Polynukleotidanteil, dadurch gekennzeichnet, dass der Linker eine Amingruppe oder ein Molekül mit mindestens zwei funktionellen Gruppen ist und mit dem lipophilen Anteil verbunden ist und der Nukleosid-, Oligonukleotid- oder Polynukleotidanteil über die funktionelle Einheit eines Phosphorsäurediesters mit dem Abstandshalter verbunden ist.

In einer bevorzugten Ausführungsform der vorliegenden Erfindung beinhalten die erfindungsgemäßen Nukleolipide eine Oligonukleotidstruktur mit 2 bis 100 Nukleotiden.

Unter Nukleinsäuren werden in der vorliegenden Erfindung sowohl Desoxy- als auch Ribonukleinsäuren verstanden. Dabei handelt es sich um Verbindungen mit einer Länge von mindestens 2, wie mindestens 4, 6, 8, 10, oder mindestens 20 oder mindestens 30 Basen.

Unter dem Ausdruck Nukleinsäuren fallen aber auch PNA Moleküle oder ähnliche, allgemein bekannte Abwandlungen von DNA oder RNA-Molekülen, wie sie im Stand der Technik bekannt sind. Die Basen, die in diesen Nukleinsäuren vorhanden sind, können dabei natürlich vorkommende Basen sein aber auch modifizierte Basen, wie sie in der Natur vorkommen oder synthetisch herstellbar sind.

Die vorliegende Erfindung betrifft insbesondere Verbindungen, reaktive Lipide, die bei Umsetzung mit einem Nukleosid, einem Oligonukleotid oder Polynukleotid ein Nukleolipid ausbilden. Diese reaktiven Lipide umfassen einen lipophilen Anteil und einen funktionellen Anteil, die über einen Linker und einen Abstandshalter miteinander verbunden sind, dadurch gekennzeichnet, dass der Linker eine Amingruppe oder ein Molekül mit mindestens zwei funktionellen Gruppen ist und mit dem lipophilen Anteil kovalent verbunden ist und der funktionelle Anteil eine Phosphoramiditgruppe ist, wobei der funktionelle Anteil über ein Sauerstoffatom mit dem Abstandshalter, der mindestens eine Kohlenstoff enthaltende Gruppe umfasst, kovalent verbunden ist.

Diese erfindungsgemäße Verbindung weist die folgende Struktur auf:

Fₙ-S- OPX₁X₂ (1)

wie in Anspruch 1 definiert.

In einer bevorzugten Ausführungsform handelt es sich dabei um eine Verbindung der allgemeinen Formel (2): 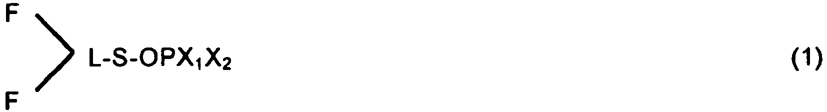 wobei F, L, S und OPX₁X₂ wie oben definiert sind.

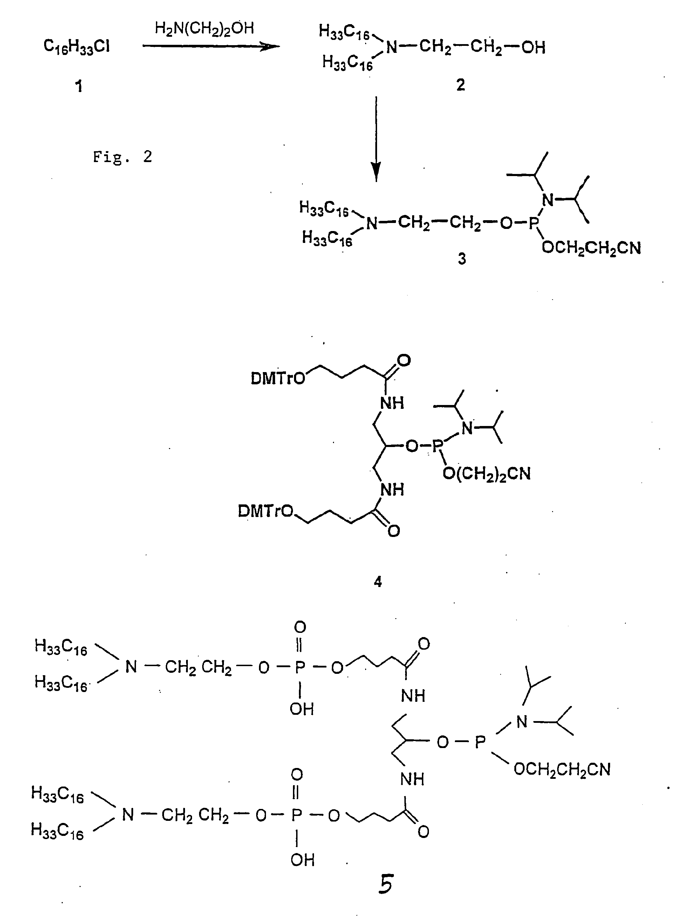

Bevorzugt weist dabei der lipophile Anteil ein Kohlenstoffgerüst mit 10 bis 40 Kohlenstoffatomen, bevorzugter mit 12 bis 40, oder mit 10 bis 20 Kohlenstoffatomen auf. Das Kohlenstoffgerüst kann ein oder mehrere konjugierte oder nicht-konjugierte Doppel- oder Dreifachbindungen enthalten. Wenn die Verbindung mehrkettige, wie doppelkettige Lipide umfasst, so können die darin enthaltenen lipophilen Anteile gleich oder verschieden sein. Der lipophile Anteil kann aber irgendein Lipid sein, die im Allgemeinen im Wasser unlöslich sind. Hier seien beispielhaft genannt: Kohlenwasserstoffe, wie Squalene und Carotinoide; Alkohol, wie Waschalkohole und Cholesterin; Ether, Carbonsäuren, gesättigte und ungesättigte Fettsäuren; Terpene und Steroide, sowie lipophile Vitamine, wie Vitamin D, E und D2. etc. Die Zahl der Lipidgruppen kann auch durch Verwendung von dendritischen Verzweigungsmolekülen weiter erhöht werden, um die Lipophilie der Verbindungen der Lipid-Nukleinsäure-Konjugate zu erhöhen (siehe z.B. Verbindung 5 in Figur 2). Die als doubling oder trebling synthons bezeichneten Verzweiger, siehe z.B. Loakes D. Organophosporous Chemistry, Vol. 33" 2003, 204ff, ermöglichen es, dass die Anzahl an lipophilen Ketten im lipophilen Anteil erhöht werden kann und somit die Lipophilie der Nukleolipide insgesamt.

Alternativ kann statt Luft durch Einsatz von z.B.Silikonöl oder anderen Ölen die Lipophilie der lipophilen Phase erhöht werden, um ein "Ziehen" durch den Nukleinsäureanteil in die hydrophile Phase zu verhindern. Es ist bevorzugt, dass die beiden Phasen des Systems eine möglichst grosse Differenz in ihren HLB-Werten aufweisen.

Die Linkergruppe ist bevorzugt ein tertiäres Amin. Eine weitere bevorzugte Linkergruppe ist ein Glycerinrest oder ein Glykolrest.

Die funktionelle Gruppe ist eine Phosphoramiditgruppe.

Eine Ausführungsform der erfindungsgemäßen Verbindung weist ein tertiäres Amin als Linker auf, eine C₂-C₈ Alkylgruppe als Abstandshalter, ein Phosphoramidit als funktionelle Gruppe, und die Lipide umfassen eine Kohlenwasser stoffkette mit 12 bis 40 Kohlenstoffatomen, wobei diese Kette(n) ein oder mehrere konjugierte und nicht-konjugierte Doppelbindungen enthalten können und wobei die Kohlenstoffketten gleich oder verschieden sein können.

In einer weiteren Ausführungsform ist der Linker ein Glykolrest, eine C₂-C₈ Alkylgruppe als Abstandshalter, ein Phosphoramidit als funktionelle Gruppe, und die Lipide umfassen eine Kohlenwasserstoffkette mit 12 bis 40 Kohlenstoffatomen, wobei diese Kette(n) ein oder mehrere konjugierte und nicht-konjugierte Doppelbindungen enthalten können und wobei die Kohlenstoffketten gleich oder verschieden sein können.

Schließlich hat eine Ausführungsform einen Glycerinrest als Linker, eine C₂-C₈ Alkylgruppe als Abstandshalter, ein Phosphoramidit als funktionelle Gruppe, und die Lipide umfassen eine Kohlenwasserstoffkette mit 12 bis 40 Kohlenstoffatomen, wobei diese Kette(n) ein oder mehrere konjugierte und nicht-konjugierte Doppelbindungen enthalten können und wobei die Kohlenstoffketten gleich oder verschieden sein können.

Mit Hilfe dieser erfindungsgemäßen Verbindungen werden nach bekannten Verfahren Nukleolipide hergestellt. Dabei umfassen bevorzugte Ausführungsformen der erfindungsgemäßen Nukleolipide solche, die mit bevorzugten Ausführungsformen der erfindungsgemäßen reaktiven Lipide hergestellt wurden. In einer besonders bevorzugten Ausführungsform ist der lipophile Anteil mit dem Linker und dem Abstandshalter über eine Phosphorsäurediestergruppe mit dem 5'-Ende des Oligo- oder Polynukleotids verbunden.

Die vorliegende Erfindung betrifft weiterhin ein Verfahren zur Herstellung von modifizierten Nukleotiden, Oligonukleotiden oder Polynukleotiden, umfassend den

Schritt des Umsetzens der erfindungsgemäßen reaktiven Lipide mit einem außer an einer OH-Gruppe geschützten Nukleosids, Oligonukleotids oder Polynukleotids. Dies erlaubt die Herstellung von Nukleolipiden, insbesondere der erfindungsgemäßen Nukleolipide.

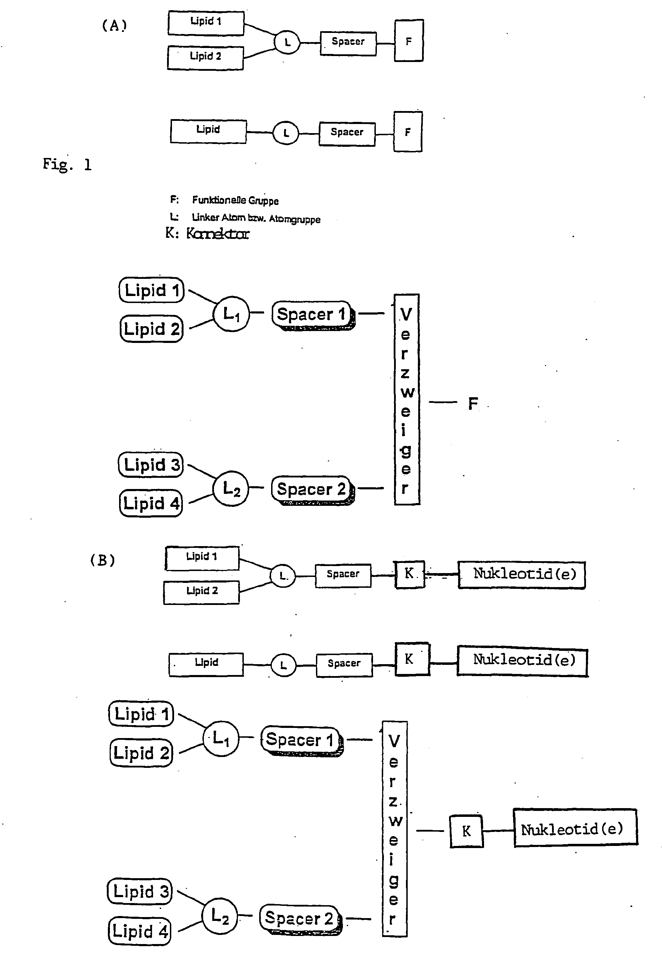

Zur Darstellung der Lipid-Probennukleinsäure-Konjugate werden zunächst in einer dem Fachmann bekannten Weise die verschiedenen Probennukleinsäure-Einzelstränge aufgebaut. Vorzugsweise geschieht dies durch automatische Festphasensynthese unter Anwendung der Phosphoramidit- bzw. der Phosphonattechnik. Der Lipidanteil wird abschließend bei der routinemäßigen automatischen Synthese als letzter Synthesebaustein mittels einer erfindungsgemäßen Verbindung, wie einem Phosphoramiditderivat oder auch einem entsprechenden Phosphonatderivat angehängt. Wie ausgeführt kann die Lipidkomponente verschiedene Strukturen besitzen, Beispiele hierfür sind in der Figur 1 dargestellt.

Die vorliegende Erfindung betrifft weiterhin ein Verfahren zum Nachweis von Nukleinsäuren. Dieses Verfahren umfasst die Schritte des Bereitstellens einer Probe, die möglicherweise Nukleinsäuren enthält, das Bereitstellen von erfindungsgemäßen Nukleolipiden, die unter hybridisierenden Bedingungen mit der nachzuweisenden Nukleinsäure hybridisiert und in Kontakt bringen der Probe mit den Nukleolipiden unter hybridisierenden Bedingungen, um ein Hybridisierungsprodukt einer Nukleinsäure aus der Probe und dem bevorzugt erfindungsgemäßen Nukleolipid auszubilden und der Nachweis des Hybridisierungsprodukts. In diesem Zusammenhang bedeutet der Ausdruck "Hybridisierung" oder "hybridisierende Bedingungen" Hybridisierung unter herkömmlichen Hybridisierungsbedingungen, insbesondere unter stringenten Bedingungen, wie sie z.B. Sambrook and Russell (2001), Molecular cloning: a laboratory manual, CSH Press. Cold Spring Harbor, N.Y., USA, beschrieben sind. In einer besonders bevorzugten Ausführungsform bedeutet der Ausdruck "Hybridisierung", dass eine Hybridisierung unter den folgenden Bedingungen erfolgt:

| | |
|---|---|
| Hybridisierungspuffer: | 2 x SSC; 10 x Denhardt-Lösung |
| | (Ficoll 400 + PEG + BSA; Verhältnis 1:1:1); 0,1 % SDS; 5 mMol EDTA; 50 mMol Na₂HPO₄; 250 µg/ml Heringssperma DNA; 50 µg/ml tRNA; oder |
| | 0,25 mol Natriumphosphatpuffer, pH 7,2; |
| | 1 mMol EDTA, |
| | 7%SDS |
| | |
| Hybridisierungstemperatur: | T = 60 °C |
| Waschpuffer: | 2 x SSC; 0,1 % SDS; |
| Waschtemperatur: | T = 60 °C |

In einer weiteren bevorzugten Ausführungsform bedeutet der Ausdruck unter hybridisierenden Bedingungen oder Hybridisierung, die Ausbildung von mehrsträngigen Hybridisierungsprodukten unter den folgenden Bedingungen:

| | |
|---|---|
| Hybridisierungspuffer: | 10 mM Na-Cacodylat, 10 mM MgCl₂, 100 mM NaCl oder 10 mM Na-Cacodylat, 100 mM MgCl₂, 1 M NaCl (letzterer zur Erhöhung von niedrigen Tm-Werten) |
| | |
| Hybridisierungstemperatur: | Raumtemperatur, individuell zwischen Raumtemperatur und 60°C in Abhängigkeit von der Länge und der Zusammensetzung der zu hybridisierenden Target- und Probensequenzen |
| Waschpuffer: | wie oben |
| Waschtemperatur: | Raumtemperatur |

In einer besonders bevorzugten Ausführungsform hybridisiert die Probensequenz mit der Targetsequenz nur bei Hybridisierungsbedingungen, bei denen die Probensequenz komplementär zu der Targetsequenz ist. Dieses ist insbesondere dann notwendig, wenn einzelne Mutationen bestimmt werden sollen, z.B. im pharmakogenetischen Bereich.

Weiterhin betrifft die vorliegende Erfindung ein Verfahren zum Nachweis des Vorhandenseins oder der Abwesenheit von Nukleinsäuren mit spezifischen Sequenzen in einer Probe umfassend die Schritte: in Kontakt bringen der Probe mit erfindungsgemäßen Nukleolipiden von Oligo- oder Polynukleotiden, wobei mindestens eine der Oligo- oder Polynukleotide eine Sequenz aufweist, die im Wesentlichen komplementär zu einer spezifischen Sequenz der Nukleinsäuren in der Probe ist und Nachweis der Ausbildung von Hybridisierungsprodukten der erfindungsgemäßen Nukleolipide und einer spezifischen Sequenz einer Nukleinsäure aus der Probe (Targetsequenz), wenn diese in der Probe vorliegt.

Die nachzuweisende Nukleinsäure mit spezifischer Sequenz (Targetsequenz) kann zuvor auf konventioneller und dem Fachmann bekannter Weise mit einer Reportergruppe markiert worden sein. Dem Fachmann sind übliche Reportergruppen oder Markermoleküle bekannt. Beispielhaft seien hier erwähnt: Fluoreszenzfarbstoffe, radioaktive Markierung, Biotin etc.

In einer bevorzugten Ausführungsform ist die Reportergruppe ein Fluoreszenzfarbstoff, z.B. Fluoreszein, ein Mitglied der Alexa-Farbstofffamilie oder der Cy-Farbstofffamilie.

In einer weiteren Ausführungsform wird das erfindungsgemäße Verfahren zum Nachweis der An- oder Abwesenheit von Nukleinsäuren mit spezifischen Sequenzen in einer Probe so durchgeführt, dass das in Kontakt bringen in mehreren voneinander abgetrennten Bereichen, wie Kompartimenten, erfolgt, wobei in den einzelnen Bereichen die Nukleolipide eine identische Nukleinsäuresequenz aufweisen, so dass in einem Bereich die vorhandenen Probensequenzen identisch sind und in jedem getrennten Bereich eine unterschiedliche Sequenz vorliegt. Alternativ können in den einzelnen Bereichen auch mehrere Nukleolipide mit unterschiedlichen vorbestimmten Nukleinsäuresequenzen vorliegen. Dieses erlaubt die Analyse mehrerer Proben.

Im Zuge der Einstellung des chemischen Gleichgewichts hybridisiert die Targetnukleinsäure an die passende Zielsequenz der Vielzahl der Probensequenzen. Gegebenenfalls kann die Hybridisierungskinetik durch Thermostatisierung der flüssigen Phase optimiert werden. Gegebenenfalls können nicht-gebundene Targetsequenzen durch einen Waschprozess entfernt werden. Bevorzugt wird die Lösung in der die Targetsequenzen vorliegen, gerührt.

Erfindungsgemäß kann der Nachweis der Hybridisierungsprodukte durch Messung der Reportergruppe erfolgen. Bei Verwendung eines Fluoreszenzfarbstoffes als Reportergruppe erfolgt die Messung der Hybridisierungsprodukte durch Messung der emittierten Fluoreszenz dieses Fluoreszenzfarbstoffes. In einer bevorzugten Ausführungsform wird dabei die Messung der Reportergruppe so durchgeführt, dass die Messung nur in dem Bereich der Flüssigkeits-Gas Grenzfläche erfolgt. In einer weiteren Ausführungsform, bei der eine zweite flüssige Phase verwendet wird, die nur gering oder überhaupt nicht mit der ersten flüssigen Phase vermischbar ist und eine Grenzfläche ausbildet, kann die Messung der Reportergruppe an der Flüssigkeit-Flüssigkeit Grenzfläche zwischen den beiden Flüssigkeiten erfolgen.

Die erfindungsgemäßen Nukleolipide eignen sich nicht nur zum Nachweis von Nukleinsäuren in Proben, sondern auch zum Isolieren von Nukleinsäuren aus einer nukleinsäurehaltigen Probe. Somit betrifft die vorliegende Erfindung weiterhin ein Verfahren zum Isolieren von Nukleinsäuren aus einer nukleinsäurehaltigen Probe, umfassend die Schritte des a) in Kontakt bringen der nukleinsäurehaltigen Probe mit Nukleolipid(en) umfassend einen lipophilen Anteil und einen Oligo- oder Polynukleotidanteil, deren Oligo- oder Polynukleotidanteil eine Hybridisierung mindestens eines Teils der Nukleinsäuren in der Probe mit dem Oligo- oder Polynukleotidanteil der Nukleolipide erlaubt und b) Abtrennen der Hybridisierungsprodukte von den übrigen Bestandteilen der Probe und gegebenenfalls Waschen der Hybridisierungsprodukte.

Bevorzugt findet das in Kontakt bringen in einer ersten flüssigen Phase statt. Durch zufügen einer zweiten flüssigen Phase, die in Verbindung mit der ersten flüssigen Phase eine flüssig-flüssig Grenzfläche ausbildet, so dass eine Spreitung an der Phasengrenze erfolgt, wobei der lipophile Anteil in die lipophilere flüssige Phase oder in der Gasphase, während der andere Teil des Nukleolipids, an dem die Nukleinsäuresequenz aus der Probe hybridisiert ist, in die andere Flüssigkeit ragt, können die Hybridisierungsprodukte von einzelsträngig vorliegenden Nukleinsäuren aus der Probe getrennt werden. Gegebenenfalls können die Hybridisierungsprodukte von den übrigen Bestandteilen der Probe abgetrennt und gegebenenfalls gewaschen werden.

In einer bevorzugten Ausführungsform des erfindungsgemäßen Verfahrens werden die Nukleolipide zur Isolierung von RNA-Molekülen, insbesondere von siRNA, miRNA oder mRNA-Molekülen verwendet. Bei der Isolierung von mRNA-Molekülen weist dabei der Nukleotidanteil der Nukleolipide eine polydT-Sequenz auf. Natürlich kann eine Isolierung auch auf Basis von anderen bekannten Sequenzen erfolgen, die in den Targetsequenzen enthalten sind.

In einer weiteren bevorzugten Ausführungsform können bestimmte Arten an Nukleinsäuren, nämlich Aptamere, für das erfindungsgemäße Isolierungsverfahren verwendet werden. Wenn Aptamere als Nukleinsäureanteil der Nukleolipide verwendet werden, ermöglicht dieses die Aufreinigung von anderen Molekülen als Nukleinsäuren, wie z.B. von Proteinen.

Weiterhin werden Kits zum Nachweis von Nukleinsäuren, die ein oder mehrere der Nukleolipide enthalten, beschrieben. Diese Kits enthalten weiterhin Anweisungen zur Durchführung des Nachweises von Nukleinsäuren und gegebenenfalls eine zweite flüssige Phase, die mit der flüssigen Probe eine Flüssig-Flüssig Grenzfläche ausbildet.

Die Nukleolipide sind weiterhin zur Herstellung von Nukleinsäurearrays geeignet. D.h., die Nukleolipide mit einem Lipidanteil und einem Oligonukleotidanteil können in Nukleinsäurearrays, sogenannten DNA-Chips, Verwendung findet. Diese DNA-Chips eignen sich z.B. zur Analyse von Genen bzw. Genabschnitten. Insbesondere für pharmakogenetische Untersuchungen sind solche Arrays geeignet. Mit Hilfe der Nukleolipide ist es nun möglich, solche Microarrays her zustellen, die im Gegensatz zu bisher beschriebenen DNA-Chips komplizierte chemische Verfahren zur Aktivierung der festen Oberfläche und zu chemischen Fixierung funktionalisierter Probensequenzen an diesen Oberflächen nicht mehr notwendig machen.

Weiterhin weisen diese Arrays den Vorteil gegenüber konventionellen Arrays mit festen, also kovalent gebundenen Nukleinsäureanteilen auf, dass eine räumliche Veränderungsmöglichkeit gegeben ist. Das heißt, bei Bildung von Hybridisierungsprodukten benötigen diese mehr Raum, so dass ein seitliches Ausweichen der Probensequenzen notwendig ist. Durch die nicht kovalente Bindung an den Träger bzw. bei einem Flüssigsystem durch die Anordnung an der Phasengrenze können die Nukleolipide seitlich ausweichen, so dass jederzeit eine optimale Dichte für die Hybridisierung vorliegt.

Somit wird in einem weiteren Aspekt ein System zur Nukleinsäureanalytik beschrieben, umfassend eine Vorrichtung, z.B. ein DNA-Chip oder einen Array, umfassend einen unteren Teil, der eine flüssige Phase aufnehmen kann und einen oberen Teil, der nicht dauerhaft mit dem unteren Teil verbunden ist und in den unteren Teil einsetzbar ist, wobei der obere Teil mindestens zwei voneinander abgetrennte Kompartimente aufweist und diese Kompartimente von der Ober- bis zur Unterseite des oberen Teils durchgehen, so dass z.B. die im unteren Teil vorhandene flüssige Phase mit der Phase in den Kompartimenten des oberen Teils darin vorhandene Targetnukleinsäuren austauschen kann.

Bevorzugt weist der obere Teil mindestens 4, 8, 16, 25, 64, 256, 384, usw. abgetrennte Kompartimente auf.

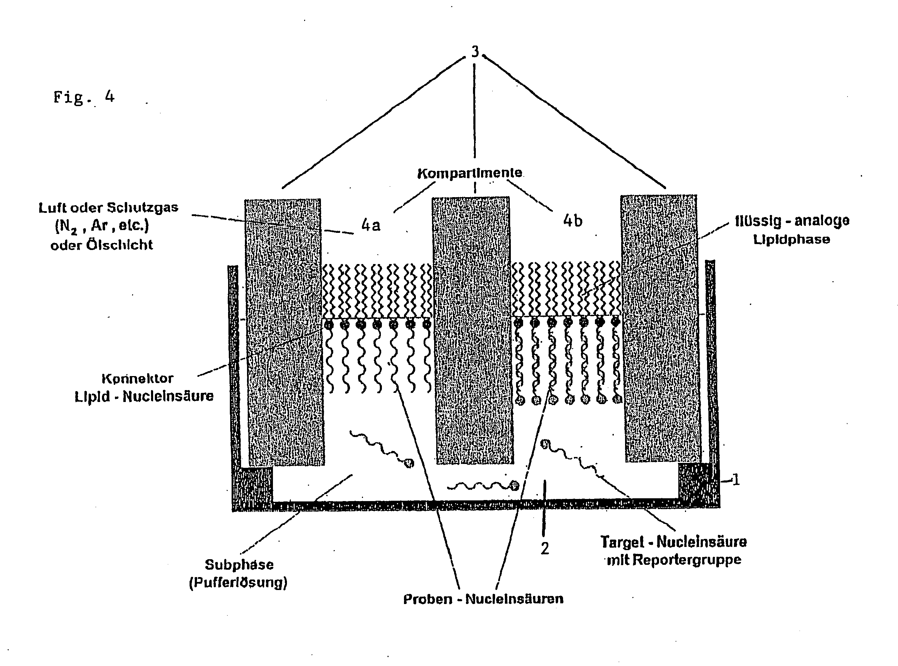

In einer bevorzugten Ausführungsform dieser Vorrichtung ist der obere Teil so ausgebildet, dass, wenn er in dem unteren Teil eingesetzt wird, der untere Abschnitt der im unteren Teil vorliegenden flüssigen Phase nicht in einzelne Kompartimente unterteilt ist. Dies ist z.B. in der Abbildung 4 dargestellt. Abbildung 4 stellt einen Querschnitt einer erfindungsgemäßen Vorrichtung dar. Der untere Teil 1 umfasst einen Bereich, in dem die erste flüssige Phase 2 vorliegt.

Der obere Teil 3 unterteilt hier den oberen Bereich der ersten flüssigen Phase in zwei Kompartimente 4a und 4b, die die erste flüssige Phase 2 enthalten. Der untere Bereich der ersten flüssigen Phase wird nicht durch den oberen Teil 3 in einzelne Kompartimente unterteilt. Hier können sich die in der ersten flüssigen Phase vorhandenen Targetnukleinsäuren mit der Reportergruppe frei bewegen. Gegebenenfalls ist eine Rührvorrichtung in diesem unteren Bereich angeordnet.

In den Kompartimenten 4a und 4b liegen die Probennukleinsäuren gespreitet an der Grenzfläche der ersten Flüssigkeit mit einem Gas oder mit der zweiten Flüssigkeit vor, wobei die Probennukleinsäuren in die erste Flüssigkeit hineinragen.

Mit Hilfe der beschriebenen Vorrichtung kann nun ein spezifischer Nachweis der Targetnukleinsäuren in Verbindung mit der Verwendung von Nukleolipiden durchgeführt werden. Im Folgenden wird beispielhaft der Ablauf einer Nukleinsäureanalytik mit Hilfe des Systems, umfassend eine oben beschriebene Vorrichtung und Nukleolipide beschrieben.

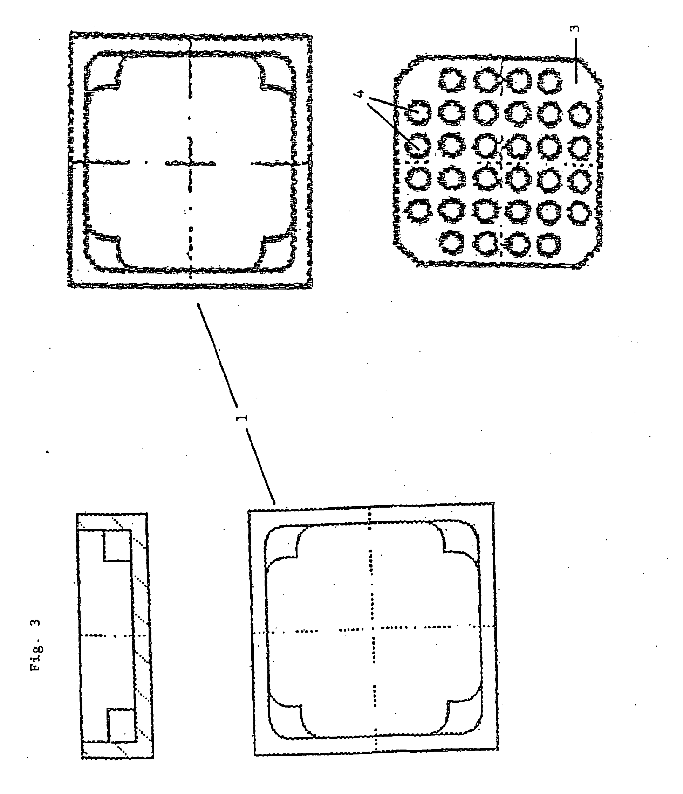

Die hoch lipophilen Oligonukleotid-Einzelstränge verschiedener Nukleotidsequenzen werden einzeln in die Kompartimente der in Fig. 3 abgebildeten Apparatur z.B. mit Hilfe eines Spotters eingebracht und bilden dort eine monomolekulare Schicht mit den Eigenschaften einer flüssig-analogen Phase. Die Lipidmoleküle weisen dabei in die Gasphase - die Oligonukleotide in die flüssige Phase (Abb. 4). Gegebenenfalls kann die wässrige Phase mit einer dünnen Ölschicht überschichtet werden, so dass die Lipidketten in die lipophile Phase weisen und sich eine flüssig-flüssig Phasengrenze ausbildet.

Die zu identifizierende Targetsequenz, die zuvor auf konventionelle und dem Fachmann bekannte Weise mit einer Reportergruppe z.B. einem Fluoreszenzfarbstoff versehen wurde, wird anschließend in die allen Probensequenzen gemeinsame Subphase injiziert und dort durch leichtes mechanisches Rühren verteilt. Im Zuge der Einstellung des chemischen Gleichgewichtes hybridisiert die Target-DNA an die passende Zielsequenz. Gegebenenfalls kann die Hybridisierungskinetik durch Thermostatisierung des unteren, die Subphase enthaltenden Teils der Apparatur und/oder durch anschließende Durchströmung der Subphase mit einer Pufferlösung (Waschprozess) optimiert werden. Im entsprechenden Kompartiment der Vorrichtung, in der die Hybridisierung zwischen optimal passender und bekannter Probensequenz und markierter Targetsequenz erfolgt, ist letztere dann mittels dem Fachmann bekannter Detektionsverfahren, z.B. mittels eines Fluoreszenzdetektors, zu identifizieren.

Die in Abb. 3 schematisch abgebildete Vorrichtung zur DNA-Analyse kann auf vielfältige Weise modifiziert werden. Die Möglichkeiten der Modifikation betreffen die Zahl, Form und Maße der Kompartimente für die Aufnahme der Probensequenzen, das Volumen der Subphase sowie das Material der Apparatur. Letzteres kann beispielsweise Plexiglas, Edelstahl oder Quarzglas sein. Zur Thermostatisierung der Subphase kann die Vorrichtung in ein Wärmebad gesetzt werden; die Temperierung kann jedoch auch auf thermoelektrischem Wege (Pelltier-Element) geschehen. Die Temperierung kann also sowohl intern als auch extern erfolgen.

In einer bevorzugten Ausführungsform umfasst die Vorrichtung eine Spottingeinheit zum Einbringen von Nukleolipiden mit spezifischer Nukleinsäuresequenz, insbesondere der Nukleolipide gemäß der vorliegenden Erfindung, in die einzelnen Kompartimente. In einer weiteren Ausführungsform umfasst die erfindungsgemäße Vorrichtung eine Einheit zur Detektion der Reportergruppen, die in den Hybridisierungsprodukten aus Targetingsequenz und Probensequenz vorhanden sind. An diese Detektionseinrichtung kann sich eine Auswerteanrichtung anschließen. Diese Anordnung erlaubt gegebenenfalls eine Echtzeitanalytik der Hybridisierungseigenschaften und eine halb-quantitative bzw. quantitative Bestimmung der Targetingsequenzen in der zu analysierenden Probe.

In einer weiteren Ausführungsform umfasst das System zur Isolierung bzw. Nachweis von Nukleinsäuren eine Vorrichtung, die ein Substrat, wie ein Willhelmy-Plättchen aus geeigneten Materialien, wie Quartzglas, reinst Silizium, TiO₂, ITO oder Materialien, die durch Beschichtung, zum Beispiel durch Silanisierung hydrophobisiert wurden, und Nukleolipide, insbesondere den beschriebenen Nukleolipiden.

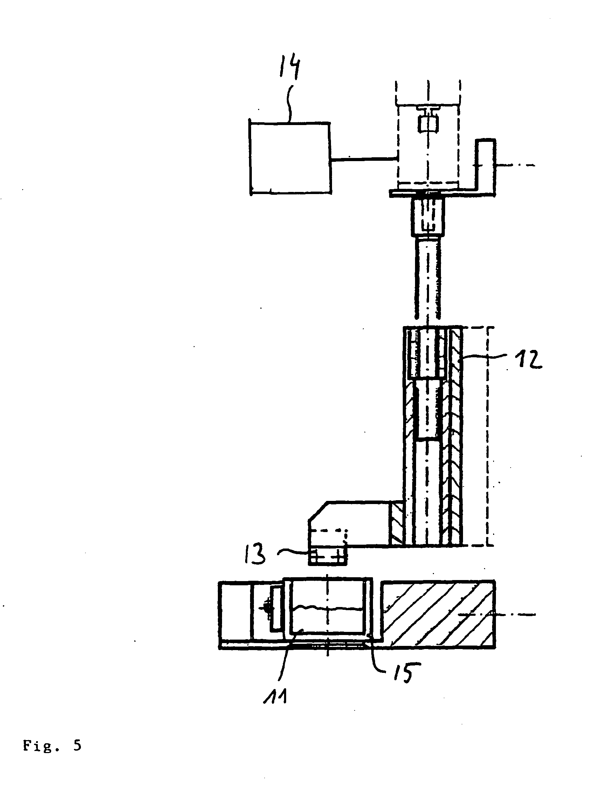

Eine solche Vorrichtung zur erfindungsgemäßen Isolierung von Nukleinsäuren ist in der Abbildung 5 dargestellt.

Die nukleinsäurehaltige Probe befindet sich in einer Vertiefung, vorliegend der Trog (1). Diese Vertiefung wird zumindest teilweise von einer Heizvorrichtung, wie einem Pelltierelement (5) oder anderen Einrichtungen zur Temperierung, wie eine Widerstandsheizung oder andere bekannte Einrichtungen umgeben, um eine Erwärmung der Flüssigkeit mit der Probe zu ermöglichen und so eine spezifische Hybridisierung zu gewährleisten. Zu der Flüssigkeit werden Nukleolipide mit vorbestimmten Nukleinsäuresequenzen gegeben, z.B. solche mit einer polyT-Sequenz zur Aufreinigung von mRNA. Die Hybridisierungsprodukte reichern sich an der Grenzfläche flüssig-gasförmig an, während nicht gebundene Nukleinsäuren aus der Probe in der Flüssigkeit frei verteilt verbleiben. Mit Hilfe einer beweglichen Vorrichtung (2), wie ein Dip-Coater, mit einem einstückig oder zweistückig angeordneten festen Träger, wie einem Plättchen, (3) können diese Hybridisierungsprodukte transferiert werden. Das heißt die Hybridisierungsprodukte lagern sich an den festen Träger an und können dann bei einem Herausziehen des Trägers von den nicht hybridisierten Nukleinsäuren der Probe getrennt werden. Als Träger oder Substrate eignen sich zum Beispiel Quartzglas, reinst Silizium, TiO₂, ITO oder Materialien, die durch Beschichtung, zum Beispiel durch Silanisierung hydrophobisiert wurden. Die lipophilen Anteile der Nucleolipide lagern sich aufgrund von nicht kovalenten Wechselwirkungen an die hydrophobe Oberfläche des Trägers an und erlauben so einen Transfer der Hybridisierungsprodukte und der einzelnen Nukleolipide. Die Steuerung des Eintauchens und Austauchen des Trägers kann dabei über eine Steuereinheit (4) erfolgen. Dabei kann das Substrat, wie das Wilhelmy-Plättchen, über einen steuerbaren Dip-Coater als Vorrichtung zur Bewegung des Trägers (2), der mit der Steuereinheit (4) verbunden ist, gesteuert werden. Das Material der Vertiefung, wie einem Trog (1), ist variabel sollte aber nicht zu hydrophob sein, um ein anhaften der Nukleolipide zu verhindern.

Bevorzugt ist also die Vorrichtung bezüglich der Ein- und Austauchgeschwindigkeit des Wilhelmy-Plättchen aber auch der Temperatur des Eintauchtroges variierbar.

Wie bereits oben ausgeführt eignen sich die erfindungsgemäßen reaktiven Lipide insbesondere zum Einsatz in herkömmlichen DNA-Synthesizern, wo sie als 5'-End-Building-Blocks eingesetzt werden können. Dies erlaubt eine einfache Synthese der Probensequenzen und vermindert die Problematik der Neosynthese der Oligonukleotide auf dem Array bzw. die schwierige chemische Fixierung vorher funktionalisierter Nukleinsäureproben auf aktivierten Oberflächen des Arrays.

Bei der Auswahl der Probennukleinsäuren besteht eine große Variationsbreite.

Hier können nicht nur aus der Natur abgeleitete DNA- und RNA-Moleküle zum Einsatz kommen. Vielmehr können auch Oligomere verwendet werden, die in mannigfaltiger Weise modifiziert sind. So kann beispielsweise zur Hybridisierung mit der zu analysierenden Target-Nukleinsäure eine komplementäre PNA (Peptid-Nukleinsäure) eingesetzt werden. Darüber hinaus wurden in den letzten Jahren verschiedene im Zuckerteil modifizierte Nukleinsäuren, z.B. Hexose- bzw. Hexitol-Nukleinsäuren, dargestellt, die zur Hybridisierung mit natürlichen Nukleinsäuren befähigt sind. Auch solche Proben-Nukleinsäuren können im hier eröffneten Analyseverfahren zum Einsatz kommen. Des weiteren wurde gezeigt, dass durch Modifikation der Nukleobasen eines Proben-Oligonukleotids, so z.B. durch Inkorporation von purin-isosteren 8-Aza-7-desaza-7-halogenopurin-Basen, die Stabilität eines Duplexes mit einem herkömmlichen DNA-Oligomer erheblich erhöht werden, und somit eine Harmonisierung der ansonsten unterschiedlich stabilen Guanin-Cytosin und Adenin-Thymin Basenpaare erreicht werden kann. Auch derartig modifizierte Proben-Nukleinsäuren können im hier eröffneten Verfahren zum Einsatz kommen.

Die Gasphase oder das Gas ist erfindungsgemäß Luft oder ein Schutzgas, wie N₂, Ar, etc. Wenn ein System mit zwei flüssigen Phasen verwendet wird, so sind diese beiden Phasen im Wesentlichen nicht mischbar und bilden eine Grenzschicht, die flüssig-flüssig Grenzfläche, aus. Dabei wird eine lipophile Phase und eine hydrophile Phase erhalten. Üblicherweise befinden sich die Targetsequenzen in der hydrophilen Phase, die üblicherweise eine wässrige Phase, wie eine Pufferlösung ist. Die lipophile Phase wird z.B. durch ein organisches Lösungsmittel oder Öl ausgebildet. Dem Fachmann sind entsprechende Systeme bekannt.

Eine weitere Möglichkeit der Verwendung von Nukleolipiden liegt in der Kennzeichnung von Zusammensetzungen, wie zum Beispiel von Rohöl oder verarbeiteten Produkten hieraus. Durch Zusatz von Nukleolipiden mit bekannter Nukleinsäuresequenz kann eine spezifische Kennzeichnung der Produkte erfolgen. Diese spezifische Kennzeichnung erlaubt eine spätere Bestimmung des Ursprungs dieser Zusammensetzungen. So können bei Ölverschmutzungen die Verursacher bestimmt werden. Durch den lipophilen Anteil ist nämlich eine Löslichkeit dieser Nukleinsäuren in Ölen und anderen lipophilen Flüssigkeiten gegeben.

Im Folgenden wird beispielhaft die Synthese einer erfindungsgemäßen Verbindung beschrieben. Es ist klar, dass die vorliegende Erfindung nicht auf die Beispiele beschränkt ist.

### Beispiel

2-Cyanoethyl-2-(dihexadecanylamino)ethyl diisopropytamidophosphan (3) 1 mmol (632.1 mg) des durch Umsetzung von 1-Chlorohexadecan mit Ethanolamin dargestellten Bis(hexadecanyl)-2-hydroxyethylamin wird in wasserfreiem Dichlormethan (25 ml) gelöst und mit Düsopropylamin (330 µl, 1.95 mmol) und Chloro-(2-cyanoethoxy)(diisopropylamino)phosphin (320 µl, 1.5 mmol) versetzt. Nach 40 minütigem Rühren bei Raumtemperatur fügt man 12 ml einer 5 proz. wässrigen NaHCO₃-Lösung hinzu und extrahiert die Mischung 3 x mit je 30 ml Dichlormethan. Die organische Phase wird getrocknet (Na₂SO₄), filtriert und eingedampft. Mitteldruckchromatographie (Kieselgel, 6 x 10 cm, 0.5 Bar, CH₂Cl₂-Aceton, 99:1) ergibt nach Eindampfen der Hauptfraktion die disastereoisomere Titelverbindung (65 %) als farblosen Feststoff. ³¹P-NMR (CDCl₃): δ 148.5, 149.0 ppm. ¹H-NMR (CDCl₃): δ 0.91 (t, J = 8 Hz, CH₃); 1,29 - 1,26 (m, CH₂); 1,51 (d, J = 4 Hz, CH₃-iPr); 2,14 (t, J = 6 Hz, CH₂); 2,32 (t, J = 7,2 Hz, CH₂-Et); 2.54 (t, J = 5.8 Hz, CH₂-N); 3.54 (m, CH₂); um 4.0 (2 m, CH₂).

## Patentansprüche

1. Verbindungen umfassend einen lipophilen Anteil und einen funktionellen Anteil, die über einen Linker (L) und einen Abstandshalter (S) miteinander verbunden sind, **dadurch gekennzeichnet, dass** der Linker eine Amingruppe oder ein Molekül mit mindestens zwei funktionellen Gruppen ist und mit dem lipophilen Anteil verbunden ist und der funktionelle Anteil eine Phosphoramiditgruppe (OPX₁X₂) ist, wobei der funktionelle Anteil über ein Sauerstoffatom mit dem Abstandshalter verbunden ist, die Verbindung hat die allgemeine Formel (1):
Fₙ-L-S-OPX₁X₂ (1)
und wobei
i) L ein tertiäres Amin ist;
n ist 2;
S ist eine C₂-C₈ Alkylgruppe;
OPX₁X₂ ist ein Phosphoramiditgruppe, wobei eine Gruppe von X₁ und X₂ eine Schutzgruppe ist und die andere Gruppe eine Abgangsgruppe bei Verbindung mit einem Nukleosid, das ein Teil eines Oligonukleotids oder Polynukleotids sein kann, ist;
F ist eine Kohlenwasserstoffkette mit 12 bis 40 Kohlenstoffatomen, wobei diese Kette ein oder mehrere konjugierte oder nicht-konjugierte Doppelbindungen enthalten können und wobei die Kohlenstoffketten gleich oder verschieden sein können;
ii) L ein Glykolrest ist;
nist 1;
S ist eine C₂-C₈ Alkylgruppe;
OPX₁X₂ ist ein Phosphoramiditgruppe, wobei eine Gruppe von X₁ und X₂ eine Schutzgruppe ist und die andere Gruppe eine Abgangsgruppe bei Verbindung mit einem Nukleosid, das ein Teil eines Oligonukleotids oder Polynukleotids sein kann, ist; und
F ist eine Kohlenwasserstoffkette mit 12 bis 40 Kohlenstoffatomen, wobei diese Kette ein oder mehrere konjugierte oder nicht-konjugierte Doppelbindungen enthalten kann; oder
iii) L ein Glycerinrest ist;
n ist 2;
S ist eine C₂-C₈ Alkylgruppe;
OPX₁X₂ ist ein Phosphoramiditgruppe, wobei eine Gruppe von X₁ und X₂ eine Schutzgruppe ist und die andere Gruppe eine Abgangsgruppe bei Verbindung mit einem Nukleosid, das ein Teil eines Oligonukleotids oder Polynukleotids sein kann, ist und
F ist eine Kohlenwasserstoffkette mit 12 bis 40 Kohlenstoffatomen, wobei diese Kette ein oder mehrere konjugierte oder nicht-konjugierte Doppelbindungen enthalten können und wobei die Kohlenstoffketten gleich oder verschieden sein können.

2. Verbindung gemäß Anspruch 1, wobei die Substituenten X₁ oder X₂ als Abgangsgruppe ausgewählt sind aus sterisch gehinderten Aminen, wie einer Diisopropylamin-Gruppe.

3. Verbindung gemäß Anspruch 1, wobei die Substituenten X₁ oder X₂ als Schutzgruppe ausgewählt sind aus einer Cyano-Gruppe enthaltenden Gruppe, wie einer Cyanoethyl-Gruppe, oder einer Methoxy-Gruppe enthaltenden Gruppe oder para-Nitrophenylethyl-Gruppe.

4. Verfahren zur Herstellung von modifizierten Nukleotiden, Oligonukleotiden oder Polynukleotiden, umfassend den Schritt des Umsetzens der Verbindungen gemäß einem der Ansprüche 1 bis 3 mit einem außer an einer OH-Gruppe geschützten Nukleosid, Oligonukleotid oder Polynukleotid unter Erhalt einer Nukleolipid-Verbindung umfassend einen lipophilen Anteil (F) und einen Nukleosid, Oligo- oder Polynukleotidanteil, die über einen Linker (L) und einen Abstandshalter (S) miteinander verbunden sind, wobei der Linker eine Amingruppe oder ein Molekül mit mindestens zwei funktionellen Gruppen und mit dem lipophilen Anteil verbunden ist und der Nukleosid, Oligo- oder Polynukleotidanteil über die funktionelle Einheit eines Phosphosäurediesters mit dem Abstandshalter verbunden ist, wobei F, L und S wie in einem der Ansprüche 1 bis 3 definiert sind.

5. Verfahren gemäß Anspruch 4, wobei die erhaltene Verbindung ein Oligonukleotid mit 2 bis 100 Nukleotiden umfasst.

6. Verfahren gemäß Anspruch 4 oder 5, wobei die funktionelle Einheit am 5'-Ende des Nukleosids, des Oligonukleotids bzw. des Polynukleotids gebunden ist.

7. Verfahren zum Nachweis von Nukleinsäuren umfassend die Schritte:
a) Bereitstellen einer Probe, die Nukleinsäuren enthält,
b) Bereitstellen von Nukleolipiden erhältlich nach einem Verfahren gemäß einem der Ansprüche 4 bis 6, deren Oligo- oder Polynukleotidanteil unter hybridisierenden Bedingungen mit der nachzuweisenden Nukleinsäure hybridisiert;
c) in Kontakt bringen der Probe gemäß a) mit dem Nukleolipiden gemäß b) unter hybridisierenden Bedingungen, um die Ausbildung eines Hybridisierungsprodukts aus einer Nukleinsäure aus der Probe und den Nukleolipiden gemäß b) zu ermöglichen;
d) Nachweis eines Hybridisierungsprodukts aus c).

8. Verfahren nach Anspruch 7 zum Nachweis des Vorhandenseins oder der Abwesenheit von Nukleinsäuren mit spezifischen Sequenzen in einer Probe umfassend die Schritte:
a) in Kontakt bringen der nukleinsäurehaltigen Probe mit Nukleolipiden umfassend einen lipophilen Anteil und einen Oligo- oder Polynukleoltidanteil, deren Oligo- oder Polynukleotidanteil eine Sequenz enthält, die im Wesentlichen komplementär zu einer spezifischen Sequenz der Nukleinsäuren in der Probe ist und unter hybridisierenden Bedingungen mit den Nukleinsäuren mit spezifischen Sequenzen hybridisiert, und den Nukleinsäuren in der Probe unter hybridisierenden Bedingungen;
b) Nachweis der Bildung von Hybridisierungsprodukten aus Nukleolipiden und einer spezifischen Sequenz einer Nukleinsäure aus der Probe.

9. Verfahren gemäß einem der Ansprüche 7 oder 8, **dadurch gekennzeichnet, dass** die Nukleinsäuren eine Reportergruppe zum Nachweis der Hybridisierungsprodukte umfasst.

10. Verfahren gemäß Anspruch 9, wobei es sich bei der Reportergruppe um einen Fluoreszenzfarbstoff handelt.

11. Verfahren gemäß einem der Ansprüche 7 bis 10, wobei der Schritt des in Kontakt bringen der Nukleinsäuren aus der Probe mit den Nukleolipiden in mindestens zwei abgetrennten Bereichen erfolgt, wobei in den einzelnen Bereichen nur Nukleolipide mit identischen Nukleosid-, Oligo- oder Polynukleotidanteil vorliegen und in den mindestens zwei getrennten Bereichen voneinander unterschiedliche Nukleolipide vorhanden sind.

12. Verfahren gemäß einem der Ansprüche 7 bis 10, wobei der Schritt des in Kontakt bringen der Nukleinsäuren aus der Probe mit den Nukleolipiden in mindestens zwei abgetrennten Bereichen erfolgt, wobei in den einzelnen Bereichen nicht miteinander mischbar unterschiedliche Proben vorliegen.

13. Verfahren gemäß einem der Ansprüche 7 bis 12 weiterhin umfassend den Schritt des Waschens der Hybridisierungsprodukte, um nicht gebundene Nukleinsäuren der Proben zu entfernen.

14. Verfahren gemäß einem der Ansprüche 7 bis 13, **dadurch gekennzeichnet, dass** der Nachweis der Hybridisierungsprodukte durch Messung der Reportergruppe, bevorzugt an einer Flüssigkeit-Gas Grenzfläche, erfolgt.

15. Verfahren gemäß einem der Ansprüche 7 bis 13, **dadurch gekennzeichnet, dass** der Nachweis der Hybridisierungsprodukte durch Messung der Reportergruppe an einer Flüssigkeit-Flüssigkeit Grenzfläche zwischen einer wässrig-hydrophilen und einer lipophilen Flüssigkeit erfolgt.

16. Verfahren nach Anspruch 7 zum Isolieren von Nukleinsäuren aus einer nukleinsäurehaltigen Probe, umfassend die Schritte des
a) in Kontakt bringen der nukleinsäurehaltigen Probe mit Nukleolipiden umfassend einen lipophilen Anteil und einen Oligo- oder Polynukleotidanteil, deren Oligo- oder Polynukleotidanteil eine Hybridisierung mindestens eines Teils der Nukleinsäuren in der Probe mit dem Oligo- oder Polynukleotidanteil der Nukleolipide erlaubt;
b) Abtrennen der Hybridisierungsprodukte von den übrigen Bestandteilen der Probe und gegebenenfalls Waschen der Hybridisierungsprodukte.

17. Verfahren gemäß Anspruch 16, wobei es zur Isolierung von RNA-Molekülen, insbesondere mRNA-Molekülen, miRNA-Molekülen und siRNA-Molekülen geeignet ist.

18. Verfahren gemäß einem der Ansprüche 16 oder 17, weiterhin umfassend den Schritt des Zufügens einer zweiten fluiden Phase, die in Verbindung mit der ersten Phase in der das in Kontakt bringen der Probe mit den Nukleolipiden erfolgt, eine Grenzfläche ausbildet, so dass der lipophile Anteil der Nukleolipide in eine der beiden Phasen ist, während sich der andere, Nukleotid-Anteil der Verbindung, an dem die Nukleinsäuren aus der Probe hybridisiert ist, in der anderen Phase vorliegt,

19. Verfahren gemäß einem der Ansprüche 16 oder 17, umfassend den Schritt der Abtrennung der Hybridisierungsprodukte mit einer geeigneten Vorrichtung, wie einem Dip-Coater mit Wilhelmy-Plättchen.

## Claims

1. Compounds comprising a lipophilic moiety and a functional moiety linked via a linker (L) and a spacer (S) with each other, **characterized in that** the linker is an amine group or a molecule having at least two functional groups and is connected with the lipophilic moiety and that the functional moiety is a phosphoramidite group (OPX₁X₂), whereby the functional moiety is connected with the spacer via an oxygen atom, the compound has the general formula (1)
Fₙ-L-S-OPX₁X₂ (1)
and whereby
i) L is a tertiary amine;
n is 2;
S is a C₂-C₈ amine group;
OPX₁X₂ is a phosphoramidite group, whereby one of the two groups X₁ and X₂ is a protecting group while the other group is a leaving group when connecting the same with a nucleoside which may be part of an oligo-nucleotide or polynucleotide;
F is a hydrocarbon chain having 12 to 40 carbon atoms, whereby the chain may have one or more conjugated or not conjugated double bonds and the carbon chains may be identical or different;
ii) L is a glycol residue;
n is 1;
S is a C₂-C₈ alkyl group;
OPX₁X₂ is a phosphoramidite group, whereby one of the two groups X₁ or X₂ is a protecting group while the other group is a leaving group when connecting the same with a nucleoside which may be part of an oligo-nucleotide or polynucleotide; and
F is a hydro-carbon chain having 12 to 40 carbon atoms, whereby the chain may have one or more conjugated or not conjugated double bonds and the carbon chains may be identical or different;
or
iii) L is a glycerol residue;
n is 2;
S is a C₂-C₈ alkyl group;
OPX₁X₂ is a phosphoramidite group, whereby one of the two groups X1 or X2 is a protecting group while the other group is a leaving group when connecting the same with a nucleoside which may be part of an oligo-nucleotide or polynucleotide; and
F is a hydro carbon chain having 12 to 40 carbon atoms, whereby the chain may have one or more conjugated or not conjugated double bonds and with said carbon atom chains may be identical or different.

2. The compound according to claim 1 wherein the substituents X₁ or X₂ being a leaving group is selected from the group of sterically hindered amines, like a diisopropylamine-group.

3. The compound according to claim 1, wherein the substituents X1 or X2 as a protecting group are selected from a cyano-group containing group, like a cyano-ethyl-group, a methoxy-group containing group, or a para-nitrophenylethyl group.

4. Method for preparation of modified nucleotides or poly-nucleotides comprising the step of reacting a compound according to any one of claims 1 to 3 with a nucleoside, oligonucleotide or polynucleotide being protected except for an OH-group to obtain a nucleolipid compound comprising a lipophilic moiety (F) and a nucleoside, oligo- or polynucleotide moiety linked via a linker (L) and a spacer (S) with each other, **characterized in that** the linker is an amine group or a molecule having at least two functional groups, and is connected with the lipophilic moiety and the nucleoside, oligo- or polynucleotide moiety is linked via a functional moiety of a phosphoric acid diester with the spacer, whereby F, L and S are defined according to any one of claims 1 to 3.

5. The method according to claim 4 whereby the compound is an oligo-nucleotide having 2 to 100 nucleotides.

6. The method according to claim 4 or 5, whereby the functional moiety is connected at the 5'-end of a nucleoside, oligonucleoside or polynucleotide, respectively.

7. A method for the detection of nucleic acids comprising the step of
a) providing a probe containing nucleic acids;
b) providing a nucleolipid obtainable according to a method according to any one of claims 4 to 6, whereby the oligo- or polynucleotides moiety hybridises with a nucleic acid to be detected under hybridising conditions;
c) contacting the probe under a) with the nucleolipids under b) under hybridising conditions allowing the formation of hybridisation products of the nucleic acid present in the sample and the nucleolipids of b);
d) detection of the hybridisation products of c).

8. The method according to claim 7 for detecting the presence or absence of nucleic acids having specific sequences in a sample comprising the steps of
a) contacting a sample containing nucleic acids with nucleolipids comprising a lipophilic moiety and a oligo- or polynucleotide moiety, whereby the oligo- or polynucleotide moiety comprises a sequence with is substantially complementary to a specific sequence of a nucleic acid present in the sample and which hybridises under hybridising conditions with a nucleic acid having a specific sequence with the nucleic acids present in the sample under hybridising conditions;
b) detecting the formation of hybridisation products of nucleolipids and a specific sequence of a nucleic acid present in the sample.

9. The method according to claim 7 or 8, **characterized in that** the nucleic acid comprises a reporter group for detection of the hybridisation product.

10. The method according to claim 9 wherein the reporter group is a fluorescence dye.

11. The method according to any one of claims 7 to 10 whereby the step of contacting the nucleic acids present in the sample with the nucleolipids is conducted in at least two individual compartments separated from each other, whereby in each of the compartments only nucleolipids with identical nucleoside oligo- or polynucleotide moieties are present and wherein in the at least two individual compartments different nucleolipids are present.

12. The method according to any one of claims 7 to 10 whereby the step of contacting the nucleic acid present in the sample with the nucleolipid is conducted in at least two compartments different from each other, whereby in the individual compartments different samples are present with are not admixable with each other.

13. The method according to any one of claims 7 to 12 further comprising the step of washing the hybridisation products to remove nucleic acids present in the probe which have not been hybridised.

14. The method according to any one of claims 7 to 13, **characterized in that** the detection of the hybridisation products is conducted by measuring the reporter group, preferably at the liquid-gas interface.

15. The method according to any one of claims 7 to 13, **characterized in that** the detection of the hybridisation products is conducted by measuring the reporter group at the liquid-liquid interface of an aqueous-hydrophilic and a lipophilic liquid occurs.

16. The method according to claim 7 for isolating nucleic acids from a sample containing nucleic acids comprising the steps of:
a) contacting the sample containing nucleic acids with nucleolipids comprising a lipophilic moiety and an oligo- or polynucleotide moiety where the oligo- or polynucleotide moiety is able to hybridise at least partly with the nucleic acids present in the sample with the oligo-or polynucleotide moiety of the nucleolipids;
b) separating the hybridisation products from the other ingredients of the sample and, optionally washing the hybridisation products.

17. The method according to claim 16 adapted for isolating RNA molecules, in particular, mRNA-molecules, miRNA-molecules and siRNA-molecules.

18. The method according to any one of claims 16 or 17 further comprising the step of adding a second liquid phase which in conjunction with the first phase in which the contact of the sample with the nucleolipids occurred an interface is formed, whereby the lipophilic moiety of the nucleolipids is in one of said phases while the nucleotide moiety of the compound to which the nucleic acids present in the probes is hybridised, is in the other phase.

19. The method according to any one of claims 16 or 17, comprising the step of separating the hybridisation products with a suitable device, like a dip-coater having a Wilhelmy-plate.

## Revendications

1. Composés comprenant une partie lipophile et une partie fonctionnelle, qui sont reliées l'une à L'autre par l'intermédiaire d'un lieur (L) et d'un espaceur (S), **caractérisés en ce que** le lieur est un groupe amino ou une molécule ayant au moins deux groupes fonctionnels et est relié à la partie lipophile, et la partie fonctionnelle est un groupe phosphoramidite (OPX₁X₂), la partie fonctionnelle étant reliée par l'intermédiaire d'un atome d'oxygène à l'espaceur, le composé ayant la formule générale (1) :
Fₙ-L-S-OPX₁X₂ (1)
et où
i) L est une amine tertiaire ;
n vaut 2 ;
S est un groupe alkyle en C₂-C₈ ;
OPX₁X₂ est un groupe phosphoramidite, dans lequel l'un des groupes X₁ et X₂ est un groupe protecteur et l'autre groupe est un groupe partant dans le cadre d'une liaison avec un nucléoside qui peut être une partie d'un oligonucléotide ou d'un polynucléotide ;
F est une chaîne hydrocarbonée ayant 12 à 40 atomes de carbone, cette chaîne pouvant contenir une ou plusieurs doubles liaisons conjuguées ou non conjuguées, et les chaînes carbonées pouvant être identiques ou différentes ;
ii) L est un résidu de glycol ;
n vaut 1 ;
S est un groupe alkyle en C₂-C₈ ;
OPX₁X₂ est un groupe phospharamidite, dans lequel l'un des groupes X₁ et X₂ est un groupe protecteur et l'autre groupe est un groupe partant dans le cadre d'une liaison avec un nucléoside qui peut être une partie d'un oligonucléotide ou d'un polynucléotide ; et
F est une chaîne hydrocarbonée ayant 12 à 40 atomes de carbone, cette chaîne pouvant contenir une ou plusieurs doubles liaisons conjuguées ou non conjuguées ; ou
iii) L est un résidu de glycérol ;
n vaut 2 ;
S est un groupe, alkyle en C₂-C₈ ;
OPX₁X₂ est un groupe phosphoramidite, dans lequel l'un des groupes X₁ et X₂ est un groupe protecteur et l'autre groupe est un groupe partant dans le cadre d'une liaison avec un nucléoside qui peut être une partie d'un oligonucléotide ou d'un polynucléotide ; et
F est une chaîne hydrocarbonée ayant 12 à 40 atomes de carbone, cette chaîne pouvant contenir une ou plusieurs doubles liaisons conjuguées ou non conjuguées, et les chaînes carbonées pouvant être identiques ou différentes.

2. Composé selon la revendication 1, dans lequel les substituants X₁ ou X₂, en tant que groupes partants, sont choisis parmi les amines à empêchement stérique, telles qu'un groupe diisopropylamine.

3. Composé selon la revendication 1, dans lequel les substituants X₁ ou X₂, en tant que groupes protecteurs, sont choisis parmi un groupe contenant un groupe cyano, tel qu'un groupe cyanoéthyle, ou un groupe contenant un groupe méthoxy, ou un groupe para-nitrophényléthyle.

4. Procédé de préparation de nucléotides, d'oligonucléotides ou de polynucléotides modifiés, comprenant l'étape de réaction des composés selon l'une des revendications 1 à 3 avec un nucléoside, un oligonucléotide ou un polynucléotide protégé par ailleurs sur un groupe OH, avec obtention d'un composé nucléolipidique comprenant une partie lipophile (F) et une partie nucléoside, oligo- ou polynucléotide, qui sont reliées l'une à l'autre par l'intermédiaire d'un lieur (L) et d'un espaceur (S), le lieur étant un groupe amino ou une molécule ayant au moins deux groupes fonctionnels et étant relié à la partie lipophile, et la partie nucléoside, oligo- ou polynucléotide étant reliée par l'intermédiaire de l'unité fonctionnelle d'un diester de l'acide phosphorique à l'espaceur, F, L et S étant définis comme dans l'une des revendications 1 à 3.

5. Procédé selon la revendication 4, dans lequel le composé obtenu comprend un oligonucléotide ayant 2 à 100 nucléotides.

6. Procédé selon la revendication 4 ou 5, dans lequel l'unité fonctionnelle est liée à l'extrémité 5' du nucléoside, de l'oligonucléotide ou du polynucléotide.

7. Procédé de détection d'acides nucléiques, comprenant les étapes de
a) mise à disposition d'un échantillon contenant des acides nucléiques,
b) mise à disposition de nucléolipides pouvant être obtenus par un procédé selon l'une des revendications 4 à 6, dont la partie oligo- ou polynucléotide s'hybride, dans des conditions d'hybridation, à l'acide nucléique à détecter ;
c) mise en contact de l'échantillon selon a) avec les nucléolipides selon b) dans des conditions d'hybridation, pour permettre la formation d'un produit d'hybridation à partir d'un acide nucléique provenant de l'échantillon et des nucléolipides selon b) ;
d) détection d'un produit d'hybridation de c).

8. Procédé selon la revendication 7 pour la détection de la présence ou de l'absence d'acides nucléiques ayant des séquences spécifiques dans un échantillon, comprenant les étapes de :
a) mise en contact de l'échantillon contenant les acides nucléiques avec des nucléolipides comprenant une partie lipophile et une partie oligo- ou polynucléotide, dont la partie oligo- ou polynucléotide contient une séquence qui pour l'essentiel est complémentaire d'une séquence spécifique des acides nucléiques se trouvant dans l'échantillon, et s'hybride dans des conditions d'hybridation aux acides nucléiques ayant des séquences spécifiques, et aux acides nucléiques se prouvant dans l'échantillon dans des conditions d'hybridation ;
b) détection de la formation de produits d'hybridation à partir des nucléolipides, et d'une séquence spécifique d'un acide nucléique provenant de l'échantillon.

9. Procédé selon l'une des revendications 7 ou 8, **caractérisé en ce que** les acides nucléiques comprennent un groupe rapporteur pour la détection des produits de l'hybridation.

10. Procédé selon la revendication 9, dans lequel, pour ce qui concerne le groupe rapporteur, il s'agit d'un colorant fluorescent.

11. Procédé selon l'une des revendications 7 à 10, dans lequel l'étape de mise en contact des acides nucléiques provenant de l'échantillon avec les nucléolipides s'effectue dans au moins deux zones distinctes, seuls des nucléolipides ayant des parties nucléosides, oligo- ou polynucléotides identiques étant présents dans les zones individuelles, et des nucléolipides différents les uns des autres étant présents dans les au moins deux zones distinctes.

12. Procédé selon l'une des revendications 7 à 10, dans lequel l'étape de mise en contact des acides nucléiques provenant de 1'échantillon avec les nucléolipides s'effectue dans au moins deux zones distinctes, les zones individuelles contenant des échantillons différents, non miscibles les uns aux autres.

13. Procédé selon l'une des revendications 7 à 12, comprenant en outre l'étape de lavage des produits de l'hybridation, pour éliminer les acides nucléiques non liés des échantillons.

14. Procédé selon l'une des revendications 7 à 13, **caractérisé en ce que** la détection des produits de l'hybridation s'effectue par mesure du groupe rapporteur, de préférence au niveau d'une interface liquide-gaz.

15. Procédé selon l'une des revendications 7 à 13, **caractérisé en ce que** la détection des produits de l'hybridation s'effectue par mesure du groupe rapporteur au niveau d'une interface liquide-liquide entre un liquide aqueux-hydrophile et un liquide lipophile.

16. Procédé selon la revendication 7, pour isoler des acides nucléiques d'un échantillon contenant des acides nucléiques, comprenant les étapes de
a) mise en contact de l'échantillon contenant des acides nucléiques avec des nucléolipides comprenant une partie lipophile et une partie oligo- ou polynucléotide, dont la partie oligo- ou polynucléotide permet une hybridation d'au moins une partie des acides nucléiques de l'échantillon à la partie oligo- ou polynucléotide des nucléolipides ;
b) séparation des produits de l'hybridation des autres constituants de l'échantillon, et éventuellement lavage des produits de l'hybridation.

17. Procédé selon la revendication 16, lequel procédé convenant à l'isolement de molécules d'ARN en particulier de molécules d'ARNm, de molécules d'ARNmi et de molécules d'ARNsi.

18. Procédé selon l'une des revendications 16 ou 17, comprenant en outre l'étape d'addition d'une deuxième phase fluide, qui, en liaison avec la première phase, dans laquelle a lieu la mise en contact de l'échantillon avec les nucléolipides, forme une interface de telle sorte que la partie lipophile des nucléolipides se trouve dans l'une des deux phases, tandis que l'autre partie, la partie nucléotide, du composé auquel s'hybrident les acides nucléiques provenant de l'échantillon, est présente dans l'autre phase.

19. Procédé selon l'une des revendications 16 ou 17, comprenant l'étape de séparation des produits de l'hybridation à l'aide d'un dispositif approprié, tel qu'une enduiseuse à immersion munie de plaques de Wilhelmy.
